# FASCICULE DE BREVET EUROPEEN

(11) **EP 3 978 039 B1**
(45) Date de publication et mention de la délivrance du brevet: **01.01.2025**
(21) Numéro de dépôt: 21200667.0
(22) Date de dépôt: 04.10.2021
(51) Int. Cl.: A61L 9/14, A61L 9/16, B60H 3/00, B61D 27/00

(54) **PROCÉDÉ ET SYSTÈME DE DÉSINFECTION PAR VOIE AÉRIENNE DE L'INTÉRIEUR D'UN VÉHICULE, NOTAMMENT UN VÉHICULE FERROVIAIRE**
VERFAHREN UND SYSTEM ZUR DESINFEKTION DES INNENRAUMS EINES FAHRZEUGS, INSBESONDERE EINES SCHIENENFAHRZEUGS, ÜBER DIE LUFT
METHOD AND SYSTEM FOR AERIAL DISINFECTION OF THE INSIDE OF A VEHICLE, IN PARTICULAR A RAILWAY VEHICLE

(30) Priorité: 05.10.2020 FR 2010143
(43) Date de publication de la demande: 06.04.2022
(73) Titulaire: SNCF Voyageurs, 93200 Saint-Denis (FR)
(72) Inventeur: GOERES, David, 72000 LE MANS (FR); PLANCHETTE, Loïc, 72100 Le Mans (FR); PUECH, Samuel, 53000 Laval (FR); JOSSET, Sébastien, 72000 Le Mans (FR)
(74) Mandataire: Fache, Sébastien

(56) Documents cités:
- CN-U- 203 697 911
- DE-A1- 102010 047 190
- FR-A1- 3 051 725
- JP-A- 2000 301 939
- JP-A- 2006 044 389

## Description

L'invention s'inscrit dans le domaine de la désinfection de véhicule.

L'invention s'inscrit plus particulièrement dans le domaine de la désinfection par voie aérienne de l'intérieur d'un véhicule de transport public, et notamment d'un véhicule ferroviaire.

Les opérations de désinfection par voie aérienne à l'intérieur des véhicules de transport, et notamment des véhicules de transport ferroviaire à destination du public, sont essentielles pour assainir l'air ambiant et éliminer les spores, virus et bactéries qui pourraient être responsables de la transmission de maladies par voie aérienne. Ces opérations sont même cruciales en période d'épidémie ou de pandémie, pour contribuer à la rupture de la chaîne de contamination.

Il est connu de désinfecter l'air intérieur de véhicule avec des produits aérosols aux propriétés désinfectantes et germicides. C'est le cas, par exemple, des documents JP 2006 044389 A, CN 203 697 911 U, JP 2000 301939 A.

Cependant, la plupart de ces véhicules de transports sont munis de moyens de ventilation commandés, et il est difficile de contrôler les flux d'air entrant et sortant du véhicule.

Il en résulte des difficultés à évaluer l'efficacité des opérations de désinfection et à doser les produits de désinfection, avec au surplus un risque d'utiliser plus de produit que nécessaire engendrant pollution et surcoût.

L'invention a pour objet de proposer un procédé de désinfection aérienne palliant les inconvénients précités.

À cet effet, l'invention vise un procédé de désinfection par voie aérienne de l'intérieur d'un véhicule, notamment un véhicule ferroviaire, lequel véhicule comprend des moyens de circulation d'air pilotés par des moyens de contrôle et de commande dudit véhicule entre un mode de circulation en circuit fermé et un mode de circulation en circuit ouvert dans lequel l'air intérieur est renouvelé avec de l'air provenant de l'extérieur du véhicule, lequel procédé comprend au moins les étapes successives de
- Actionnement du mode de circulation fermée par les moyens de contrôle et de commande ;
- Relargage dans l'air intérieur du véhicule d'un produit aérosol ;
- Lancement d'une minuterie durant une durée déterminée par les moyens de contrôle et de commande, pendant laquelle les moyens de contrôle maintiennent le mode de circulation en circuit fermé, l'air intérieur étant ainsi mélangé au désinfectant recirculant dans le véhicule, et
- A l'issue de l'écoulement la durée déterminée, évacuation du produit aérosol par basculement vers le mode de circulation ouverte par les moyens de contrôle et de commande. - Lancement d'une seconde minuterie durant une seconde durée déterminée par les moyens de contrôle et de commande concomitamment à l'étape d'évacuation du produit aérosol pendant laquelle les moyens de contrôle maintiennent le mode de circulation en circuit ouvert.

Le procédé peut également comporter les caractéristiques optionnelles suivantes considérées isolément ou selon toutes les combinaisons techniques possibles :
- Le véhicule comprend au moins deux compartiments séparés par au moins une porte pilotée par les moyens de contrôle et de commande, lesquels moyens de contrôle et de commande pilotent l'ouverture de ladite porte préalablement au relargage du produit aérosol et la maintiennent ouverte au moins jusqu'à l'issue de l'écoulement de la seconde durée déterminée précédant l'évacuation du produit aérosol.
- Le relargage du produit aérosol est piloté par les moyens de contrôle et de commande.
- Le procédé comprend une étape préalable de fermeture d'au moins une porte d'accès depuis l'extérieur du véhicule.
- Le procédé comprend une étape de génération et d'envoi d'au moins un message indiquant l'état d'avancement du procédé de désinfection en cours.

L'invention vise également un système de désinfection par voie aérienne de l'intérieur d'un véhicule, notamment un véhicule ferroviaire, comprenant des moyens de circulation d'air prévus pour être installé dans le véhicule et pilotés par des moyens de contrôle et de commande dudit système entre un mode de circulation en circuit fermé et un mode de circulation en circuit ouvert, et un récipient étanche de stockage d'un produit aérosol relié fluidiquement aux moyens de circulation d'air par l'intermédiaire d'un moyen de régulation piloté par les moyens de contrôle et de commande qui sont configurés pour mettre en oeuvre un procédé de désinfection comprenant au moins les étapes successives décrites ci-dessus.

L'invention vise également un véhicule de transport ferroviaire comprenant des moyens de circulation d'air installés dans le véhicule et pilotés par des moyens de contrôle et de commande dudit véhicule entre un mode de circulation en circuit fermé et un mode de circulation en circuit ouvert dans lequel l'air intérieur est renouvelé avec de l'air provenant de l'extérieur du véhicule, et un récipient étanche configuré pour stocker un produit aérosol et relié fluidiquement aux moyens de circulation d'air par l'intermédiaire d'un moyen de régulation piloté par les moyens de contrôle et de commande qui sont configurés pour mettre en oeuvre un procédé de désinfection comprenant au moins les étapes successives décrites ci-dessus.

D'autres caractéristiques et avantages de l'invention ressortiront clairement de la description qui en est donnée ci-dessous, à titre indicatif et nullement limitatif, en référence aux figures annexées :

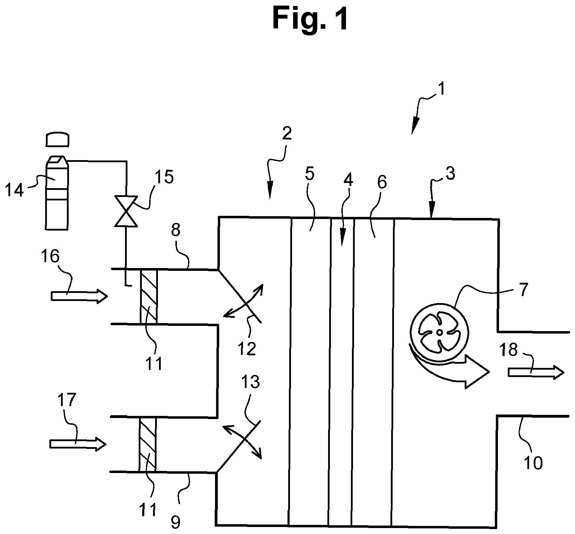

[Fig 1] la figure 1 représente une vue schématique d'un système de désinfection aérienne selon l'invention.

Il est précisé que la figure représente essentiellement un mode de réalisation de l'objet de l'invention mais qu'il peut exister d'autres modes de réalisation qui répondent à la définition de l'invention.

L'invention concerne un procédé de désinfection par voie aérienne de l'intérieur d'un véhicule, et plus particulièrement d'un véhicule de transport public du type véhicule ferroviaire.

Un tel véhicule ferroviaire comprend classiquement au moins une voiture destinée au transport de voyageurs dites voiture voyageur, et comprend préférentiellement une pluralité de ces voitures voyageurs. Les voitures voyageurs sont classiquement séparées par des portes intérieures dont l'ouverture et la fermeture sont pilotés par des moyens de contrôle et de commande du véhicule. Ces portes intérieures séparent ainsi les voitures voyageurs en plusieurs compartiments.

Le véhicule ferroviaire comprend également des portes d'accès depuis l'extérieur du véhicule, lesquelles portes d'accès sont pilotées en ouverture et en fermeture par les moyens de contrôle et de commande.

Ces moyens de contrôle et de commande comprennent des moyens informatiques électriquement reliés à une interface homme machine, laquelle interface comprend au moins un dispositif d'affichage de type écran et au moins un organe de commande configuré pour déclencher, ou le cas échéant stopper, le procédé de désinfection selon l'invention. De manière avantageuse, le dispositif d'affichage est un écran tactile et les moyens informatiques génèrent l'affichage d'icônes de commande permettant à un opérateur de déclencher ou le cas échéant de stopper, par interaction avec l'écran tactile, le procédé de désinfection de l'invention.

En référence à la figure 1, le véhicule ferroviaire comprend des moyens de circulation d'air 2 formés par des moyens de ventilation 3 et de climatisation 4 de l'air intérieur 18. Ces moyens de ventilation 3 comprennent classiquement au moins un premier conduit 8, dit conduit interne 8, assurant la circulation de l'air en circuit fermé 16. En d'autres termes, ce premier conduit 8 ne communique pas avec l'extérieur du véhicule. Les moyens de ventilation 3 comprennent en outre au moins un second conduit 9, dit conduit externe 9, assurant la circulation d'air 17 depuis l'extérieur du véhicule vers l'intérieur du véhicule. Ce second conduit 9 assure donc le renouvellement de l'air intérieur 18 du véhicule ferroviaire.

De manière avantageuse, au moins un filtre 11 est installé à l'intérieur de chaque conduit 8, 9 de manière à filtrer l'air 16, 17 circulant dans lesdits conduits 8, 9. Les moyens de ventilation 3 comprennent en outre au moins un premier volet de réglage, dit volet interne 12, piloté par les moyens de contrôle et de commande entre une position d'ouverture du conduit interne 8, permettant à l'air 16 de recirculer dans ce conduit interne 8, et une position de fermeture dudit conduit interne 8, empêchant toute circulation d'air via ce conduit 8. Les moyens de ventilation 3 comprennent également au moins un second volet de réglage, dit volet externe 13, piloté par les moyens de contrôlé et de commande entre une position d'ouverture du conduit externe 9, permettant à l'air provenant de l'extérieur 17 de circuler dans ce conduit externe 9, et une position de fermeture dudit conduit externe 9, empêchant toute circulation d'air via ce conduit 9.

Les moyens de ventilation 3 comprennent également un dispositif de ventilation du type ventilateur 7 piloté par les moyens de contrôle et de commande. En outre, les moyens de circulation 2 comprennent des moyens de climatisation de l'air intérieur 4 comprenant au moins une batterie froide 5 de stockage de frigories et une batterie chaude 6 de stockage de calories, lesdites batteries froide et chaude 5, 6 étant disposées dans le flux d'air 16, 17 provenant des conduits interne 8 ou externe 9 pour refroidir ou réchauffer ledit flux d'air 16, 17.

Les moyens de ventilation 3 comprennent enfin au moins un conduit d'évacuation formant une sortie d'air 10 débouchant à l'intérieur du véhicule, laquelle sortie d'air 10 est fluidiquement reliée au conduit interne 8 et au conduit externe 9.

De manière connue, les moyens de contrôle et de commande sont aptes à mettre en oeuvre au moins un programme de gestion des moyens de ventilation 3, qui assure le pilotage des différents éléments des moyens de ventilation 3 et notamment des volets 12, 13. En particulier, ce programme de gestion assure le basculement entre un mode de circulation en circuit fermé et un mode de circulation en circuit ouvert.

Dans le mode de circulation en circuit fermé, le volet interne 12 est ouvert et le volet externe 13 est fermé : l'air intérieur 18 circule de manière étanche via le conduit interne 8 des moyens de ventilation 3 sans être mélangé à l'air extérieur 17. Dans le mode de circulation ouvert, le volet interne 12 est fermé et le volet externe 13 est ouvert : l'air intérieur 18 est renouvelé avec de l'air extérieur 17 circulant via le conduit externe 9. Dans tous les cas, le ventilateur 7 est actionné pour permettre la circulation d'air 16, 17 dans les conduits 8, 9 et sa sortie dans l'habitacle interne du véhicule via la sortie d'air 10.

Ainsi les moyens de contrôle et de commande, via le programme de gestion, assurent le basculement entre le mode de circulation fermé, privilégié lorsque l'air intérieur 18 doit être amené et maintenu à une température désirée en limitant les dépenses énergétiques, et le mode de circulation ouvert, privilégié lorsque l'air intérieur 18 doit être assaini et renouvelé. Dans la suite de la description, ce mode de fonctionnement mis en oeuvre par le programme de gestion est appelé mode de fonctionnement nominal.

L'invention permet à un opérateur, via les moyens de contrôle et de commande, d'agir sur ce programme de gestion des moyens de ventilation 3 pour assurer une désinfection optimale de l'air intérieur 18 du véhicule, via un procédé de désinfection par voie aérienne qui va maintenant être décrit.

Au cours d'un première étape, l'opérateur lance le programme de désinfection via l'interface homme machine. Un message sous forme de pictogramme indiquant que le procédé de désinfection est en cours s'affiche sur le dispositif d'affichage de l'interface homme machine. De manière avantageuse, un autre message est envoyée via les moyens de contrôle et de commande vers un dispositif d'affichage extérieur situé en dehors du véhicule, par exemple sur un quai, précisant que le véhicule est en cours de maintenance et que la montée y est interdite.

Au cours d'une deuxième étape, les moyens de contrôle et de commande pilotent le basculement (ou le cas échéant le maintien) des moyens de ventilation 3 vers le mode de circulation fermée.

Dans un premier mode de réalisation dit mode semi-manuel, l'opérateur dépose à l'intérieur du véhicule, au cours d'une troisième étape, un récipient contenant un produit aérosol désinfectant. L'opérateur ouvre alors le récipient pour permettre le relargage du produit aérosol dans l'air intérieur 18 du véhicule puis quitte ledit véhicule.

De manière alternative et dans un second mode de réalisation dit automatique, les moyens de circulation d'air 2 comprennent un récipient étanche 14 fluidiquement relié au conduit interne 8 des moyens de ventilation 3 par l'intermédiaire d'un moyen de régulation 15, par exemple une vanne automatique pilotée par les moyens de contrôle et de commande. Le récipient étanche 14 est rempli de produit aérosol désinfectant. Au cours de la troisième étape, les moyens de contrôle et de commande pilotent l'ouverture de la vanne automatique 15, induisant le relargage du produit aérosol dans l'air intérieur 18 du véhicule.

Les moyens de circulation d'air 2, les moyens de contrôle et de commande et le récipient étanche 14 rempli de produit désinfectant aérosol et relié aux moyens de ventilation 3 forment un système de désinfection 1 de l'air intérieur 18 selon l'invention.

Une première minuterie d'une durée déterminée et enregistrée dans un espace mémoire des moyens informatiques est alors lancée par les moyens de contrôle et de commande, durant laquelle l'air intérieur 18 mélangé au désinfectant en aérosol recircule par le conduit interne 8 des moyens de ventilation 3, ce qui a pour effet de répartir de manière homogène le produit aérosol dans l'air intérieur 18 du véhicule. Cela a également pour effet de désinfecter le conduit interne 8 ainsi que les moyens de ventilation 3 et les moyens de climatisation 4 en même temps que l'air intérieur 18. La durée de la première minuterie peut être paramétrée par l'opérateur. Dans tous les cas, la première minuterie est programmée pour assurer la désinfection optimale de l'air intérieur 18, et l'élimination des germes et virus initialement présents dans l'air intérieur 18 du véhicule.

De manière à rendre plus homogène et plus efficace la désinfection de l'air intérieur 18, les moyens de contrôle et de commande pilotent, préalablement ou concomitamment à l'étape de relargage de l'aérosol désinfectant, l'ouverture des portes intérieures de compartiments et la fermeture des portes d'accès. Par ailleurs, les moyens de contrôle et de commande maintiennent les portes intérieures ouvertes et les portes d'accès fermées.

En parallèle, les moyens de contrôle et de commande envoient un message vers le dispositif d'affichage de l'interface homme machine, et le cas échéant vers le dispositif d'affichage extérieur, indiquant que la désinfection avec le produit aérosol est en cours.

A l'issue de la première durée déterminée de la première minuterie, c'est-à-dire une fois la durée déterminée écoulée, les moyens de contrôle et de commande pilotent le basculement des moyens de ventilation 3 du mode de circulation fermée vers le mode de circulation ouvert, ce qui permet un renouvellement de l'air intérieur 18 et l'évacuation complète du produit désinfectant vers l'extérieur du véhicule. Concomitamment, l'air chargé d'aérosol désinfectant et circulant dans le conduit d'évacuation 10 assure la désinfection dudit conduit 10.

Une seconde minuterie est alors lancée par les moyens de contrôle et de commande, dont la durée est déterminée et enregistrée dans l'espace mémoire des moyens informatiques. A l'instar de la première minuterie, la seconde minuterie peut être paramétrée par l'opérateur. Dans tous les cas, la seconde minuterie est programmée pour assurer le renouvellement complet de l'air intérieur 18 et l'évacuation totale du produit désinfectant.

Parallèlement, les moyens de contrôle et de commande envoient un message vers le dispositif d'affichage de l'interface homme machine, et le cas échéant vers le dispositif d'affichage extérieur, indiquant que la désinfection de l'air intérieur 18 avec le produit aérosol est terminée et que le renouvellement de l'air intérieur 18 est en cours. Durant cette seconde minuterie, il n'est pas possible de relarguer de nouveau du produit désinfectant dans l'air intérieur 18, ni de faire basculer les moyens de ventilation 3 vers le mode de circulation fermé.

A l'issue de la seconde durée déterminée de la seconde minuterie, c'est-à-dire une fois la seconde durée déterminée écoulée, les moyens de contrôle et de commande mettent fin au procédé de désinfection et relancent le programme de gestion assurant le mode de fonctionnement nominal des moyens de circulation d'air 2.

Les moyens de contrôle et de commande envoient alors un message sur le dispositif d'affichage de l'interface homme machine indiquant le retour en mode de fonctionnement nominal. Le cas échéant, un autre message est envoyer par les moyens de contrôle et de commande vers le dispositif d'affichage externe pour indiquer la fin du procédé de désinfection et la fin de l'interdiction de monter à bord du véhicule.

De manière avantageuse, les portes intérieures ne sont plus maintenues ouvertes par les moyens de contrôle et de commande. En outre, les portes d'accès au véhicule sont débloqués par les moyens de contrôle et de commande.

## Revendications

1. Procédé de désinfection par voie aérienne de l'intérieur d'un véhicule, notamment un véhicule ferroviaire, lequel véhicule comprend des moyens de circulation d'air (2) pilotés par des moyens de contrôle et de commande dudit véhicule entre un mode de circulation en circuit fermé dans lequel l'air intérieur (18) circule sans être mélangé à de l'air provenant de l'extérieur (17) du véhicule et un mode de circulation en circuit ouvert dans lequel l'air intérieur (18) est renouvelé avec de l'air (17) extérieur, lequel procédé comprend au moins les étapes successives de :
• Actionnement du mode de circulation fermée par les moyens de contrôle et de commande ;
• Relargage dans l'air intérieur (18) du véhicule d'un produit aérosol ;
• Lancement d'une minuterie durant une durée déterminée par les moyens de contrôle et de commande pendant laquelle les moyens de contrôle maintiennent le mode de circulation en circuit fermé, l'air intérieur (18) mélangé au désinfectant recirculant dans le véhicule ;
• A l'issue de l'écoulement de la durée déterminée, évacuation du produit aérosol par basculement vers le mode de circulation ouverte par les moyens de contrôle et de commande, et
• Lancement d'une seconde minuterie durant une seconde durée déterminée par les moyens de contrôle et de commande concomitamment à l'étape d'évacuation du produit aérosol pendant laquelle les moyens de contrôle maintiennent le mode de circulation en circuit ouvert.

2. Procédé selon la revendication 1, **caractérisé en ce que** le véhicule comprend au moins deux compartiments séparés par au moins une porte pilotée par les moyens de contrôle et de commande, lesquels moyens de contrôle et de commande pilotent l'ouverture de ladite porte préalablement au relargage du produit aérosol et la maintiennent ouverte au moins jusqu'à l'issue de l'écoulement de la seconde durée déterminée précédant l'évacuation du produit aérosol.

3. Procédé selon la revendication 1 ou 2, **caractérisé en ce que** le relargage du produit aérosol est piloté par les moyens de contrôle et de commande.

4. Procédé selon l'une quelconque des revendications précédentes, **caractérisé en ce qu'**il comprend une étape préalable de fermeture d'au moins une porte d'accès depuis l'extérieur du véhicule.

5. Procédé selon l'une quelconque des revendications précédentes, **caractérisé en ce qu'**il comprend une étape de génération et d'envoi d'au moins un message indiquant l'état d'avancement du procédé de désinfection en cours.

6. Système de désinfection (1) par voie aérienne de l'intérieur d'un véhicule, notamment un véhicule ferroviaire, comprenant des moyens de circulation d'air (2) prévus pour être installé dans le véhicule et pilotés par des moyens de contrôle et de commande dudit système entre un mode de circulation en circuit fermé et un mode de circulation en circuit ouvert, et un récipient étanche (14) de stockage d'un produit aérosol relié fluidiquement aux moyens de circulation d'air (2) par l'intermédiaire d'un moyen de régulation (15) piloté par les moyens de contrôle et de commande, **caractérisé en ce que** les moyens de contrôle et de commande sont configurés pour mettre en oeuvre un procédé de désinfection comprenant au moins les étapes successives de :
• Actionnement du mode de circulation fermée par les moyens de contrôle et de commande ;
• Relargage dans l'air intérieur (18) du véhicule d'un produit aérosol ;
• Lancement d'une minuterie durant une durée déterminée par les moyens de contrôle et de commande pendant laquelle les moyens de contrôle maintiennent le mode de circulation en circuit fermé, l'air intérieur (18) mélangé au désinfectant recirculant dans le véhicule ;
• A l'issue de l'écoulement de la durée déterminée, évacuation du produit aérosol par basculement vers le mode de circulation ouverte par les moyens de contrôle et de commande, et
• Lancement d'une seconde minuterie durant une seconde durée déterminée par les moyens de contrôle et de commande concomitamment à l'étape d'évacuation du produit aérosol pendant laquelle les moyens de contrôle maintiennent le mode de circulation en circuit ouvert.

7. Véhicule de transport ferroviaire comprenant des moyens de circulation d'air (2) installés dans le véhicule et pilotés par des moyens de contrôle et de commande dudit véhicule entre un mode de circulation en circuit fermé et un mode de circulation en circuit ouvert dans lequel l'air intérieur (18) est renouvelé avec de l'air (17) provenant de l'extérieur du véhicule, et un récipient étanche (14) configuré pour stocker un produit aérosol et relié fluidiquement aux moyens de circulation d'air par l'intermédiaire d'un moyen de régulation (15) piloté par les moyens de contrôle et de commande, **caractérisé en ce que** les moyens de contrôle et de commande sont configurés pour mettre en oeuvre un procédé de désinfection par voie aérienne de l'intérieur du véhicule comprenant au moins les étapes successives de :
• Actionnement du mode de circulation fermée par les moyens de contrôle et de commande ;
• Relargage dans l'air intérieur (18) du véhicule d'un produit aérosol ;
• Lancement d'une minuterie durant une durée déterminée par les moyens de contrôle et de commande pendant laquelle les moyens de contrôle maintiennent le mode de circulation en circuit fermé, l'air intérieur (18) mélangé au désinfectant recirculant dans le véhicule ;
• A l'issue de l'écoulement de la durée déterminée, évacuation du produit aérosol par basculement vers le mode de circulation ouverte par les moyens de contrôle et de commande, et
• Lancement d'une seconde minuterie durant une seconde durée déterminée par les moyens de contrôle et de commande concomitamment à l'étape d'évacuation du produit aérosol pendant laquelle les moyens de contrôle maintiennent le mode de circulation en circuit ouvert.

## Patentansprüche

1. Verfahren zur Desinfektion des Inneren eines Fahrzeugs, insbesondere eines Schienenfahrzeugs, über die Luft, wobei das Fahrzeug Luftzirkulationsmittel (2) umfasst, die von Kontroll- und Steuermitteln des Fahrzeugs zwischen einem Zirkulationsmodus im geschlossenen Kreis, in dem die Innenluft (18) zirkuliert, ohne mit Luft von außerhalb (17) des Fahrzeugs vermischt zu werden, und einem Zirkulationsmodus im offenen Kreis, in dem die Innenluft (18) mit Außenluft (17) erneuert wird, gesteuert werden, wobei das Verfahren wenigstens die folgenden aufeinanderfolgenden Schritte umfasst:
• Betätigen des Modus der geschlossenen Zirkulation durch die Kontroll- und Steuermittel;
• Abgeben eines Aerosolprodukts in die Innenluft (18) des Fahrzeugs;
• Starten eines Timers während einer durch die Kontroll- und Steuermittel bestimmten Zeitdauer, während der die Kontrollmittel den Zirkulationsmodus im geschlossenen Kreis beibehalten, wobei die Innenluft (18), die mit dem Desinfektionsmittel vermischt ist, im Fahrzeug rezirkuliert;
• Nach Ablauf der bestimmten Zeitdauer, Abführen des Aerosolprodukts durch Umschaltung zum Modus der offenen Zirkulation durch die Kontroll- und Steuermittel, und
• Starten eines zweiten Timers während einer durch die Kontroll- und Steuermittel bestimmten zweiten Zeitdauer, gleichzeitig zum Schritt des Abführens des Aerosolprodukts, während welcher die Kontrollmittel den Zirkulationsmodus im offenen Kreis beibehalten.

2. Verfahren nach Anspruch 1, **dadurch gekennzeichnet, dass** das Fahrzeug wenigstens zwei Abteile umfasst, die durch wenigstens eine Tür getrennt sind, die von den Kontroll- und Steuermitteln gesteuert wird, wobei die Kontroll- und Steuermittel die Öffnung der Tür vor der Abgabe des Aerosolprodukts steuern und sie wenigstens bis zum Ablauf der bestimmten zweiten Zeitdauer, die der Abführung des Aerosolprodukts vorangeht, offen halten.

3. Verfahren nach Anspruch 1 oder 2, **dadurch gekennzeichnet, dass** die Abgabe des Aerosolprodukts von den Kontroll- und Steuermitteln gesteuert wird.

4. Verfahren nach einem der vorhergehenden Ansprüche, **dadurch gekennzeichnet, dass** es einen vorhergehenden Schritt des Schließens wenigstens einer Tür zum Zugang von außerhalb des Fahrzeugs umfasst.

5. Verfahren nach einem der vorhergehenden Ansprüche, **dadurch gekennzeichnet, dass** es einen Schritt des Erzeugens und Sendens wenigstens einer Meldung umfasst, die den Fortschritt des laufenden Desinfektionsverfahrens angibt.

6. System zur Desinfektion (1) des Inneren eines Fahrzeugs, insbesondere eines Schienenfahrzeugs, über die Luft, umfassend Luftzirkulationsmittel (2), die dafür vorgesehen sind, im Fahrzeug eingebaut zu sein und von Kontroll- und Steuermitteln des Systems zwischen einem Zirkulationsmodus im geschlossenen Kreis und einem Zirkulationsmodus im offenen Kreis gesteuert zu werden, und einen dichten Behälter (14) zur Speicherung eines Aerosolprodukts, der mittels eines Regelungsmittels (15), das von den Kontroll- und Steuermitteln gesteuert wird, fluidisch mit den Luftzirkulationsmitteln (2) verbunden ist, **dadurch gekennzeichnet, dass** die Kontroll- und Steuermittel dazu ausgebildet sind, ein Desinfektionsverfahren durchzuführen, das wenigstens die folgenden aufeinanderfolgenden Schritte umfasst:
• Betätigen des Modus der geschlossenen Zirkulation durch die Kontroll- und Steuermittel;
• Abgeben eines Aerosolprodukts in die Innenluft (18) des Fahrzeugs;
• Starten eines Timers während einer durch die Kontroll- und Steuermittel bestimmten Zeitdauer, während der die Kontrollmittel den Zirkulationsmodus im geschlossenen Kreis beibehalten, wobei die Innenluft (18), die mit dem Desinfektionsmittel vermischt ist, im Fahrzeug rezirkuliert;
• Nach Ablauf der bestimmten Zeitdauer, Abführen des Aerosolprodukts durch Umschaltung zum Modus der offenen Zirkulation durch die Kontroll- und Steuermittel, und
• Starten eines zweiten Timers während einer durch die Kontroll- und Steuermittel bestimmten zweiten Zeitdauer, gleichzeitig zum Schritt des Abführens des Aerosolprodukts, während welcher die Kontrollmittel den Zirkulationsmodus im offenen Kreis beibehalten.

7. Schienentransportfahrzeug umfassend Luftzirkulationsmittel (2), die im Fahrzeug eingebaut sind und von Kontroll- und Steuermitteln des Fahrzeugs zwischen einem Zirkulationsmodus im geschlossenen Kreis und einem Zirkulationsmodus im offenen Kreis, in dem die Innenluft (18) mit Außenluft (17) von außerhalb (17) des Fahrzeugs erneuert wird, gesteuert werden, und einen dichten Behälter (14), der dazu ausgebildet ist, ein Aerosolprodukt zu speichern und mittels eines Regelungsmittels (15), das von den Kontroll- und Steuermitteln gesteuert wird, fluidisch mit den Luftzirkulationsmitteln (2) verbunden ist, **dadurch gekennzeichnet, dass** die Kontroll- und Steuermittel dazu ausgebildet sind, ein Verfahren zur Desinfektion des Inneren des Fahrzeugs über die Luft durchzuführen, das wenigstens die folgenden aufeinanderfolgenden Schritte umfasst:
• Betätigen des Modus der geschlossenen Zirkulation durch die Kontroll- und Steuermittel;
• Abgeben eines Aerosolprodukts in die Innenluft (18) des Fahrzeugs;
• Starten eines Timers während einer durch die Kontroll- und Steuermittel bestimmten Zeitdauer, während der die Kontrollmittel den Zirkulationsmodus im geschlossenen Kreis beibehalten, wobei die Innenluft (18), die mit dem Desinfektionsmittel vermischt ist, im Fahrzeug rezirkuliert;
• Nach Ablauf der bestimmten Zeitdauer, Abführen des Aerosolprodukts durch Umschaltung zum Modus der offenen Zirkulation durch die Kontroll- und Steuermittel, und
• Starten eines zweiten Timers während einer durch die Kontroll- und Steuermittel bestimmten zweiten Zeitdauer, gleichzeitig zum Schritt des Abführens des Aerosolprodukts, während welcher die Kontrollmittel den Zirkulationsmodus im offenen Kreis beibehalten.

## Claims

1. Method for aerial disinfection of the interior of a vehicle, in particular a railway vehicle, which vehicle comprises air-circulation means (2) controlled by command and control means of said vehicle between a closed-circuit circulation mode, in which the interior air (18) circulates without being mixed with air (17) coming from the exterior of the vehicle, and an open-circuit circulation mode, in which the interior air (18) is refreshed with exterior air (17), which method comprises at least the successive steps of:
• actuating the closed-circulation mode by the command and control means,
• releasing an aerosol product into the interior air (18) of the vehicle,
• starting a timer for a predetermined period by the command and control means, during which period the control means maintain the closed-circuit circulation mode, the interior air (18) mixed with the disinfectant recirculating inside the vehicle,
• after the expiry of the predetermined period, evacuating the aerosol product by switching to the open-circulation mode by the command and control means, and
• starting a second timer for a second predetermined period by the command and control means concomitantly with the step of evacuating the aerosol product, during which period the control means maintain the open-circuit circulation mode.

2. Method according to Claim 1, wherein the vehicle comprises at least two compartments separated by at least one door controlled by the command and control means, which command and control means command said door to open prior to releasing the aerosol product and keep it open at least until after the expiry of the second predetermined period preceding the evacuation of the aerosol product.

3. Method according to Claim 1 or 2, wherein the release of the aerosol product is controlled by the command and control means.

4. Method according to any one of the preceding claims, wherein it comprises a prior step of closing at least one access door for access from the exterior of the vehicle.

5. Method according to any one of the preceding claims, wherein it comprises a step of generating and sending at least one message indicating the state of progression of the disinfection method in progress.

6. System (1) for aerial disinfection of the interior of a vehicle, in particular a railway vehicle, comprising air-circulation means (2) provided to be installed in the vehicle and controlled by command and control means of said system between a closed-circuit circulation mode and an open-circuit circulation mode, and a sealed storage container (14) for an aerosol product that is fluidically connected to the air-circulation means (2) via a regulating means (15) controlled by the command and control means, wherein the command and control means are configured to implement a disinfection method comprising at least the successive steps of:
• actuating the closed-circulation mode by the command and control means,
• releasing an aerosol product into the interior air (18) of the vehicle,
• starting a timer for a predetermined period by means of the command and control means, during which period the control means maintain the closed-circuit circulation mode, the interior air (18) mixed with the disinfectant recirculating inside the vehicle,
• after the expiry of the predetermined period, evacuating the aerosol product by switching to the open-circulation mode by the command and control means, and
• starting a second timer for a second predetermined period by the command and control means concomitantly with the step of evacuating the aerosol product, during which period the control means maintain the open-circuit circulation mode.

7. Railway transport vehicle comprising air-circulation means (2) installed in the vehicle and controlled by command and control means of said vehicle between a closed-circuit circulation mode and an open-circuit circulation mode in which the interior air (18) is refreshed with air (17) coming from the exterior of the vehicle, and a sealed container (14) configured to store an aerosol product and fluidically connected to the air-circulation means via a regulating means (15) controlled by the command and control means, wherein the command and control means are configured to implement a method of aerial disinfection of the interior of the vehicle comprising at least the successive steps of:
• actuating the closed-circulation mode by the command and control means,
• releasing an aerosol product into the interior air (18) of the vehicle,
• starting a timer for a predetermined period by the command and control means, during which period the control means maintain the closed-circuit circulation mode, the interior air (18) mixed with the disinfectant recirculating inside the vehicle,
• after the expiry of the predetermined period, evacuating the aerosol product by switching to the open-circulation mode by the command and control means, and
• starting a second timer for a second predetermined period by the command and control means concomitantly with the step of evacuating the aerosol product, during which period the control means maintain the open-circuit circulation mode.
